# EUROPEAN PATENT APPLICATION

(11) **EP 2 505 214 A1**
(43) Date of publication of application: **03.10.2012**
(21) Application number: 11002683.8
(22) Date of filing: 31.03.2011
(51) Int. Cl.: A61L 15/32, A61L 15/42

(54) **A tissue engineering scaffold**

(71) Applicant: University of Brighton, Brighton, East Sussex BN2 4AT (GB)
(72) Inventor: James, Stuart Laurence, East Sussex, BN25 3SB (GB); Shevchenko, Rotislav Vladimirovich, Brighton, BN2 8FZ (GB)
(74) Representative: Schlich, George William

(57) **Abstract**

The invention provides a tissue engineering scaffold for application to a wound, the tissue engineering scaffold comprising a tissue regeneration matrix layer (1) for contacting the wound and a protective backing element (2) bonded to the tissue regeneration matrix layer, the tissue regeneration matrix layer (1) being formed from a porous biodegradable material capable of taking up cellular material from the wound and supporting tissue generation from the cellular material, and the protective backing element (2) being formed from a water-impermeable material; wherein the protective backing element is provided with at least one cavity (4) therein containing an aqueous medium, the channel or cavity (4) being in fluid communication with the tissue regeneration matrix layer so that one or more biomarker substances from the tissue regeneration matrix layer can diffuse into the aqueous medium the cavity;
and wherein the protective backing element (2) is configured to allow aqueous medium to be withdrawn from the cavity for analysis.

## Description

This invention relates to a tissue engineering scaffold for application to a wound and in particular to wounds of large surface area such as burns and chronic ulcers where substantial tissue regeneration is required.

### Background of the Invention

Large-area full-thickness burns have been treated successfully for over two decades using a tissue engineering approach employing a variety of scaffolds manufactured from biodegradable materials. Numerous biodegradable scaffolds for guided tissue restoration have been described and reviewed. [see Shevchenko, James and James, 2010]. An important requirement of wound dressings comprising biodegradable scaffolds is that the material from which the scaffold is formed needs to be incorporated into the wound quickly without causing an excessive inflammatory reaction and needs to degrade with time. Consequently, natural polymers are generally more likely to be suitable for the purpose than synthetic polymers. Collagen has proved to be the material of a choice for biodegradable scaffolds production due to its excellent biocompatibility properties and collagen is widely used for bioengineered tissue reconstruction purposes. Collagen gels and fibres reconstituted from purified collagen are ideal for artificial implantable scaffolds because of their low inflammatory and antigenic responses which make these materials suitable even for a vascular reconstruction. [Ravi et al., 2009]

One biodegradable scaffold in widespread use is the Integra Dermal Regeneration Template which comprises a porous dermal component made of bovine type I collagen and shark chondroitin-6-sulphate glycosaminoglycan which is bonded to a silicone pseudo-epidermis. [Yannas and Burke, 1980] This material was successfully clinically tested in managing burn wounds in 1981 and since then has become widely adopted for full-thickness burns treatment. Advantages of the product include its long shelf life, simple handling, low risks of immunogenic response, good cosmetic outcomes with reduced rates of contraction and scarring and excellent tolerance by the host tissues.

Clinically, the regeneration process is usually monitored experimentally by the surgeon, noting aspects such as colour, degree of granulation and contraction. Numerous attempts have been made to quantify the variables, using near infra red spectroscopy to monitor water content, multiple-spectroscopy to measure oxygen tension and sidestream dark-field imaging to monitor angiogenesis. However, such methods are sophisticated and expensive in terms of equipment and trained operators. Other approaches have involved direct sampling, such as taking punch biopsies. Although this is useful in a research context, such invasive procedures in a routine clinical environment are ill advised, particularly because of the possibility of infection, but also because intervention in the healing template might compromise the process. Attempts at direct sampling of wound exudates have been reported, although not in the presence of a tissue scaffold. Foremost among the techniques for sampling wound exudates in real-time and over prolonged periods have been microdialysis. It has been applied to cerebral spinal fluid, muscle and adipose tissue, and also to skin. It has been used invasively and semi-invasively (i.e. laid on to the wound bed) and not in the presence of a tissue regeneration scaffold. It is also important that such probes cannot be incorporated into the regeneration tissue. Additionally, this technology can be expensive in the context of industrial production if it to be routinely incorporated into a commercially manufactured scaffold. Therefore, there exists a need for a means of acquiring key parameters indicating successful regeneration of the dermal layer which is inexpensive, easy to use, robust and can generate data in real-time both in a research context, and subsequently in the clinical environment as a routinely manufactured product. Although a variety of attempts have been made at modifying wound dressings so that they can report the healing status of wounds, such as chronic ulcers and surgical wounds, considerably less is known about the biochemical signature of a successfully regenerating tissue scaffold in the clinical situation. Several decades ago, wound pH was measured as an indicator of healing, it is thus surprising that even now, not enough is known about this variable to use it as an indicator of successful tissue regeneration. Similarly, oxygen tension in a healing ulcer is reported as having to be greater than 30 mm Hg for successful healing but the same measurements in a successful regeneration environment are not yet established

### Summary of the Invention

It has now been found that by providing a channel containing an aqueous medium in the backing layer of a tissue engineering scaffold such as the silicone backed biodegradable gelatine gel scaffolds described above, and by arranging the channel so that it is in fluid contact with the collagen gel, molecules associated with the healing of a wound diffuse into the aqueous medium in the channel. The aqueous medium containing these "biomarker substances" can then be drawn off though a port connected to the channel and subsequently analysed. By analysing the collected aqueous medium for the reporter substances, the status of the wound can be determined.

Accordingly, in a first aspect, the invention provides a tissue engineering scaffold for application to a wound, the tissue engineering scaffold comprising a tissue regeneration matrix layer for contacting the wound and a protective backing element bonded to the tissue regeneration matrix layer, the tissue regeneration matrix layer being formed from a porous biodegradable material capable of taking up cellular material from the wound and supporting tissue generation from the cellular material, and the protective backing element being formed from a water-impermeable material;
wherein the protective backing element is provided with at least one cavity therein containing an aqueous medium, the channel or cavity being in fluid communication with the tissue regeneration matrix layer so that one or more biomarker substances from the tissue regeneration matrix layer can diffuse into the aqueous medium the cavity;
and wherein the protective backing element is configured to allow aqueous medium to be withdrawn from the cavity for analysis.

The cavity may be, for example, a channel which is connected to a port.

Accordingly, in one embodiment, the invention provides a tissue engineering scaffold for application to a wound, the tissue engineering scaffold comprising a tissue regeneration matrix layer for contacting the wound and a protective backing element bonded to the tissue regeneration matrix layer, the tissue regeneration matrix layer being formed from a porous biodegradable material capable of taking up cellular material from the wound and supporting tissue generation from the cellular material, and the protective backing element being formed from a water-impermeable material;
wherein the protective backing element is provided with at least one channel therein, the channel being connected to a port through which an aqueous medium can be introduced into the channel or withdrawn therefrom, and wherein the channel is in fluid communication with the tissue regeneration matrix layer so that one or more biomarker substances from the tissue regeneration matrix layer can diffuse into aqueous medium present in the channel and subsequently be withdrawn though the port for analysis.

Alternatively, the cavity may take the form of a bubble which can be accessed by syringe needle to extract the aqueous medium. In this embodiment, the protective backing element is formed from a self-sealing material such as a silicone which can be penetrated with a syringe needle but can reform a liquid-tight seal after the needle has been withdrawn.

Acordingly, in another embodiment, the invention provides a tissue engineering scaffold for application to a wound, the tissue engineering scaffold comprising a tissue regeneration matrix layer for contacting the wound and a protective backing element bonded to the tissue regeneration matrix layer, the tissue regeneration matrix layer being formed from a porous biodegradable material capable of taking up cellular material from the wound and supporting tissue generation from the cellular material, and the protective backing element being formed from a water-impermeable material;
wherein the protective backing element is provided with at least one cavity therein containing an aqueous medium, the channel or cavity being in fluid communication with the tissue regeneration matrix layer so that one or more biomarker substances from the tissue regeneration matrix layer can diffuse into the aqueous medium the cavity;
and wherein the protective backing element is formed from a self sealing material through which a syringe needle can be pushed so that aqueous medium can be withdrawn from the cavity for analysis.

The tissue regeneration matrix layer serves as a scaffold in which tissue regeneration can take place. Cells from the wound migrate into the matrix and grow and reproduce to form tissue, protected by the protective backing element. As new tissue grows, so the matrix layer gradually breaks down and is absorbed by the new tissue.

Once healing is complete, the protective backing element can be peeled away from the healed wound and regenerated tissue.

Growth of new tissue is accompanied by the release of various biomarker substances such as matrix metalloproteinases, tissue inhibitory metalloproteinases and proinflammatory cytokines.

Matrix metalloproteinases (MMPs) are zinc-dependent endopeptidases which are involved in a number of biological processes such as cell proliferation, migration (adhesion/dispersion), differentiation and angiogenesis. Matrix metalloproteinases are capable of degrading extracellular matrix proteins such as collagen.

Proinflammatory cytokines include interleukins such as IL-6 which is abundantly produced and secreted by epithelial cells.

Monitoring levels of selected biomarkers provides a means of assessing the status of a wound and the extent of healing that has taken place. Biomarkers produced by the regenerating tissue diffuse into the channels in the protective element which in use are filled with an aqueous medium such as physiological saline solution. The aqueous medium containing the biomarkers can then be withdrawn from the channels through the ports and subjected to analysis by standard methods to determine the types and quantities of the biomarkers.

The tissue regeneration matrix layer is formed from a porous biodegradable material capable of taking up cellular material from the wound. The biodegradable material should be biocompatible and typically therefore is of natural origin. The biodegradable material may be in the form of a gel, for example a gel formed from cross-linked protein material such as collagen or gelatine.

Alternatively, the matrix layer may be formed from or contain a biocompatible synthetic matrix or may comprise a mixture of materials of natural origin and biocompatible synthetic materials.

In one particular embodiment, the biodegradable material comprises a cross-linked gelatine gel.

In another embodiment, the biodegradable material comprises a cross-linked collagen gel.

The cross-linking agent used to form the gel can be any cross-linking agent conventionally used to form protein gels. Examples include glutaradehyde and dialdehyde starch. One preferred cross-linking agent is glutaraldehyde.

The tissue regeneration matrix layer (e.g. cross-linked protein gel) is porous, the pores in the material enabling it to take up cellular materials from the wound. Thus the pore sizes should be sufficient to enable cells to migrate into the matrix and grow and reproduce to form tissue.

The tissue regeneration matrix layer preferably comprises a cryogel. Cryogels are formed by cross-linking gel-forming protein materials such as collagen or gelatine at temperatures below 0 °C, more usually below -10 °C. The formation of ice crystals in the gel-forming mixtures gives rise to the formation of large interconnected pores so that the resulting gel has a somewhat spongy nature. Methods of gelatine cryogelation are described in Dainiak et al., "Gelatin-fibrinogen cryogel dermal matrices for wound repair", Biomaterials, 31: 67-76, 2010.

The protective backing element is formed from a water-impermeable material which provides mechanical protection for the wound, and protection from pathogenic microorganisms as well as preventing the tissue regeneration matrix from drying out.

In one embodiment, the protective backing element comprises a flexible and mouldable polymeric sheet.

Preferably the protective backing element comprises a silicone polymer and more preferably the protective backing layer is formed predominantly from a silicone polymer. The silicone polymer is typically present as a layer of silicone which covers the tissue regeneration matrix layer. The layer of silicone may be unilaminar or multilaminar. For example, it may be formed from a single integrally formed layer of silicone or from a plurality (e.g. two) of layers of silicone bonded together.

The protective backing element or layer has at least one cavity (e.g. channel or bubble) therein which is in fluid communication with the tissue regeneration matrix layer. The cavity (e.g. channel or bubble) may be creating by cutting or carving a layer of the water impermeable material (e.g. the silicone) but more typically it is formed by casting the material (e.g. silicone) in a suitably shaped mould.

Where the cavity is a channel, typically it has two ends and a pair of inlet/outlet openings, one at each end thereof. The openings may take the form of ports or may be connected (e.g. via short lengths of tubing) to ports. The ports permit an aqueous to be introduced into the channel and subsequently withdrawn from the channel when required. The ports are preferably provided with closure means that can be opened or closed to allow or prevent access to the channels. The closure means can be, for example, a removable cap or bung placed over or in the port. Alternatively, the closure can take the form of a valve or tap-like arrangement. The closure means can take the form of a luer-lok (TM) or a luer connector or cap.

The channels may be tubular in form, with the tubes having one or more holes permitting fluid communication with the tissue regeneration matrix layer, or the channels may be open on one side (a side facing the tissue regeneration matrix) to permit the necessary fluid communication. In a further alternative, the channels may be partially lined with tubes. Where the channels are formed by or incorporate tubes, the tubes may be made from a silicone material.

In one embodiment, the protective backing layer is formed from silicone and has embedded therein a tube formed from a silicone material. The tube has a number of holes or windows cut into it which permit fluid communication with the tissue regeneration matrix layer.

The protective backing element is bonded to the tissue regeneration matrix layer. The strength of the bond must be such that the backing element and matrix layer do not peel apart during normal use. Bonding may be achieved by virtue of intrinsic adhesion between the backing layer and matrix layer but more typically the surface of the backing element will need to be modified to provide adhesion or an adhesive such as a medical grade cyanoacrylate adhesive may be used. In one embodiment, when the protective backing element is formed from a silicone material, a primer or surface modifying agent is applied to the silicone to improve its ability to adhere to the tissue regeneration matrix layer. Examples of such primer or surface modifying agents are aminoalkyltrialkoxysilanes such as aminopropyltriethoxysilane.

The term "wound" as used herein refers generally to a location on a body where tissue regeneration may be required. Thus, for example, the wound may be an area on the external surface of the body where there has been extensive trauma resulting in loss of large areas of dermal tissue. The wound may be, for example, a burn, e.g. a full thickness burn or chronic ulceration. The tissue engineering scaffold may therefore take the form of a wound dressing for application to an external surface of the body.

The tissue engineering scaffolds of the invention enable the healing progress to be monitored without the need to disturb the wound before healing has reached a sufficiently advanced stage.

The channel in the protective backing element or layer is filled with an aqueous medium which is physiologically compatible. The aqueous medium is typically isotonic. An example of such an aqueous medium is physiological saline. After the scaffold has been in place on the wound for a defined period, aqueous medium is withdrawn from the channel (for example by syringe needle where the cavity is a bubble or by means of a syringe attached to a luer fitting on the ports when the cavity is a channel). The aqueous medium is then tested for the presence of defined biomarkers such as the MMPs and inflammatory cytokines described above. The methods used for testing and quantifying the biomarkers can be conventional methods. For example, in the case of MMPs and proinflammatory cytokines such as IL-6, identification and quantification can be by means of a specific Enzyme Linked Immunosorbent Assay (ELISA) which may be purchased commercially or constructed from individual components.

The tissue engineering scaffolds of the invention can be used in a research sense to investigate the levels of biomarkers associated with particular stages of healing. For example, the scaffolds may be applied to test animals such as pigs and the appearance of selected biomarkers over a period of time monitored. In this way, information and data about the levels of selected biomarkers can be collected and applied in a clinical situation.

In a further aspect, the invention provides a method of monitoring the progress of healing of a wound, which method comprises one or more cycles, each of which cycles comprises the steps applying to the wound a tissue engineering scaffold as defined herein, filling the cavity with an aqueous medium (preferably a physiologically acceptable aqueous medium such as physiological saline), and where necessary closing any ports to prevent loss of the aqueous medium, leaving the aqueous medium in the channel for a defined period, withdrawing the aqueous medium from the cavity, and testing the aqueous medium to identify and optionally quantify one or more selected biomarkers in the aqueous medium.

Following removal of the aqueous medium, the cavity (e.g. channel or bubble) may be refilled with fresh aqueous medium, optionally with an intermediate flushing step, and the method repeated in a fresh cycle. The number of cycles and the length of the defined period may depend on *inter alia* the nature of the wound and the expected rate of healing. For example, the defined period (i.e. the period the aqueous medium resides in the channel) would typically be from 30 minutes to about 12 hours. The cycle could be repeated on a daily basis (or such other frequency as is considered necessary) until the supervising medical practitioner is satisfied that the wound is healing well.

The invention will now be described in more detail, but not limited, by reference to the specific embodiments illustrated in the accompanying drawings Figures 1 to 16

### Brief Description of the Drawings

Figure 1 is a schematic side sectional elevation of a tissue engineering scaffold according to one embodiment of the invention.
Figure 2 is a view from above of the scaffold of Figure 1, but with the embedded regions of the tube shown by means of dashed lines.
Figure 3 is a schematic side sectional elevation of a tissue engineering scaffold according to a second embodiment of the invention.
Figure 4 is a view from above of the scaffold of Figure 1, but with the embedded regions of the tube and the outlines of the channels shown by means of dashed lines.
Figure 5 is a schematic side sectional elevation of a tissue engineering scaffold according to a third embodiment of the invention.
Figure 6 is a view from above of the scaffold of Figure 1, but with the sampling bubble shown by means of a dashed line.
Figures 7 and 8 show the percentage recovery of copper sulphate via the sampling channels in the Embodiments of Examples 1 and 2 when the scaffolds are immersed in copper sulphate solution.
Figure 9 shows the percentage recovery of methyl orange via the sampling channels in the Embodiment of Example 1 when the scaffold is immersed in methyl orange solution.
Figures 10 and 11 show the percentage recovery of blue dextran via the sampling channels in the Embodiments of Examples 1 and 2 when the scaffolds are immersed in blue dextran solution.
Figure 12 show the percentage recovery of Fast Green FCF via the sampling channels in the Embodiments of Examples 1 and 2 when the scaffold is immersed in Fast Green FCF solution.
Figures 13 and 14 show the percentage recovery of bovine serum albumin via the sampling channels in the Embodiments of Examples 1 and 2 when the scaffolds are immersed in bovine serum albumin solution.
Figure 15 shows the percentage recovery of IL-6 via the sampling channels in the Embodiments of Example 1 with no fibroblast cells added to the scaffold.
Figure 16 shows the percentage recovery of IL-6 via the sampling channels in the Embodiments of Example 1 after fibroblast cells have been seeded into the scaffold.

### Detailed Description of the Invention

### EXAMPLE 1

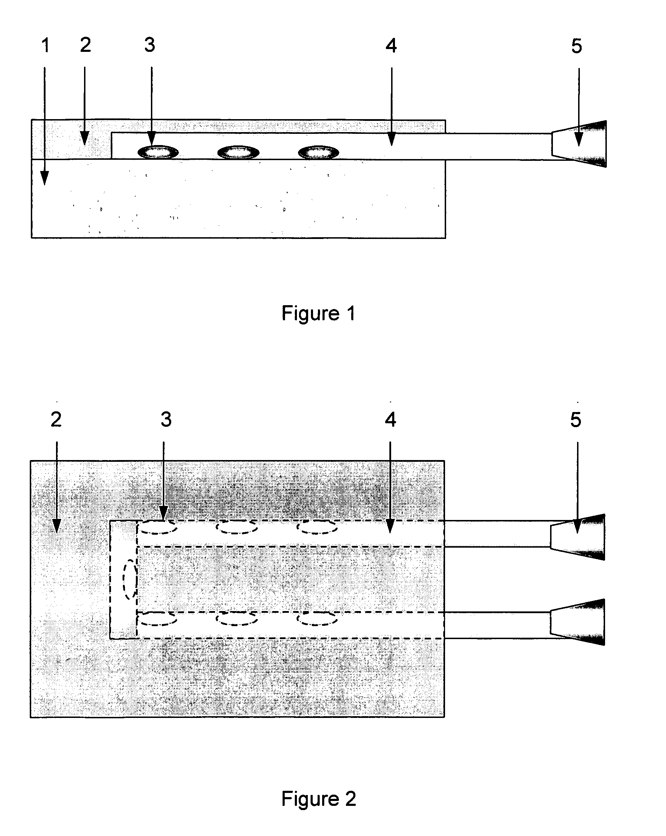

Figures 1 and 2 illustrate a tissue engineering scaffold according to one embodiment of the invention. In this embodiment, the scaffold comprises a tissue regeneration matrix layer 1 formed from a gelatine cryogel and a protective backing element or layer 2 formed from a silicone material. Embedded in the silicone layer 2 is a length of silicone tubing 4 which terminates at each end with ports 5. The ports are provided with removable resealable closures (not shown).

The tissue engineering scaffold of this embodiment can be applied to a wound such as a burn and left in place while healing of the wound takes place. At regular intervals (e.g. daily or twice weekly) the ports are opened and aqueous medium is withdrawn from the channel formed by the tubing. The aqueous medium is then subjected to analysis to identify and quantify biomarker substances such as matrix metalloproteinases and proinflammatory cytokines which are indicative of wound healing.

Once healing of the wound is judged to be complete, the silicone backing payer may be peeled off to reveal regenerated tissue (e.g. skin).

The scaffold of Figures 1 and 2 is prepared as follows.

Liquid silicone (Sylgard 184, Dow Corning, USA) is cast into an appropriately shaped glass mould to produce a layer 1 mm thick and is polymerised at 80°C for 20 minutes.

A length of silicone tubing (normal wall translucent silicone 1 mm x 1 mm, BS2775, Fisherbrand, UK) is placed over the polymerised silicone layer and shaped to form a loop. The ends of the tubing extend beyond the silicone backing layer.

Liquid silicone (Sylgard 184, Dow Corning, USA) is further cast into the glass mould to produce a layer 2-3mm thick to encapsulate the length of silicone tubing. The silicone pre-polymer is polymerised at 80⁰C for 60 minutes.

The surface of the silicone backling layer is modified with aminopropyltriethoxysilane (APTES) solution to improve protein immobilisation. The silicone backing layer is immersed in 5% APTES v/v in ethanol overnight at room temperature (5% v/v APTES (3-aminopropyltriethoxysilane, AcroSeal 99%, Acros Organics, Fisher Scientific, UK), 5% H₂O v/v and 90% v/v ethanol (Ethanol, absolute 99%, Fisher Scientific, UK)).

Ports for syringe attachment are glued to the ends of the silicone tubing using cyanoacrylate glue with activator (LOCTITE All Plastics, Henkel, UK. Adhesive base: ethyl cyanoacrylate, activator solvent: heptane).

Wound fluid collection windows (x5, 1x3mm) are cut into the walls of the silicone tubing on the side where the wound healing scaffold matrix is planned to be attached.

The tubing is filled with water and frozen for 1 hour to prevent cryogel solution entering and polymerising within the tubing.

Aqueous gelatin solution 6%w/w (Gelatin from cold water fish skin,~45% in H₂O, G7765, Sigma, UK), crosslinked with 0.4%v/v glutaraldehyde (Glutaraldehyde solution, Grade II, 25% in H₂O, G6257, Sigma-Aldrich, UK), is prepared, cast over the silicone backing element and polymerised using a cryopolymerisation process (Julabo ethanol cryobath, -12°C) for 20 hours.

The silicone-cryogel sandwich with incorporated tubing is thawed out at room temperature, removed from the mould and washed with distilled water (x3 times).

To block residual glutaraldehyde within the scaffold, the silicone-cryogel sandwich with incorporated tubing is immersed into 0.5M glycine (glycine, G7126, Sigma, UK) 50mM phosphate-buffered solution (sodium phosphate diabasic, S0876, Sigma, UK) for 20 hours at +4°C.

The scaffold is then washed with distilled water (x3 times) followed by phosphate buffered saline (PBS tablets, BR014G, Oxoid, UK) (x3 times).

The finished scaffold is sealed in polyethylene double sachets with 5ml of PBS to avoid drying out and sterilised with gamma-irradiation (>25kGy, Isotron Ltd., UK).

### EXAMPLE 2

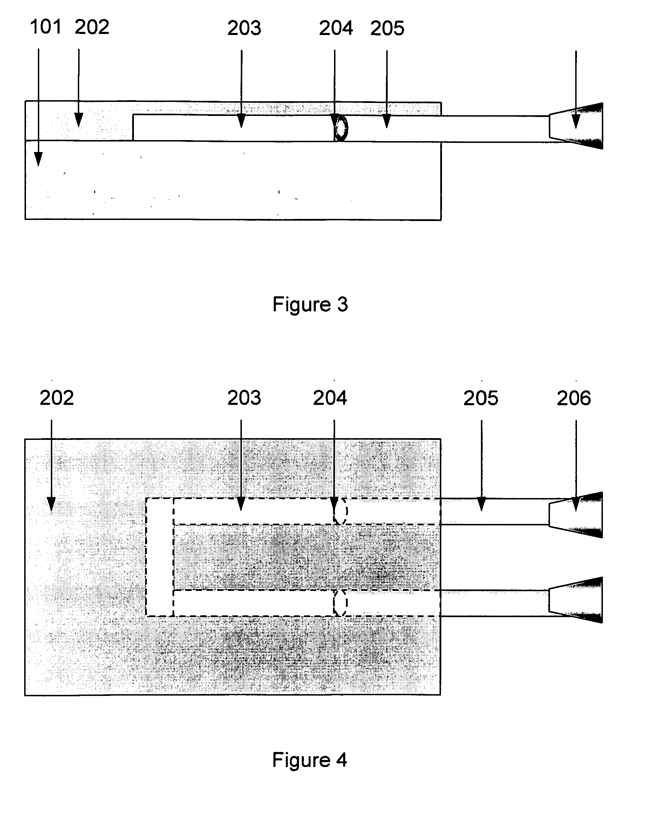

Figures 3 and 4 illustrate a tissue engineering scaffold according to a second embodiment of the invention. In this embodiment, the scaffold comprises a tissue regeneration matrix layer 101 formed from a gelatine cryogel and a protective backing element or layer 102 formed from a silicone material. Embedded in the silicone layer 102 are short lengths of silicone tubing 105 each of which terminates with a port 106. The ports are provided with removable resealable closures (not shown). The inner ends 104 of the lengths of silicone tubing open out into a U-shaped channel 3 which is open on its lower side, i.e. the side facing the gelatine cryogel matrix.

The tissue engineering scaffold of Figures 3 and 4 may be used in essentially the same way as the scaffold of Figures 1 and 2.

The scaffold of Figures 3 and 4 is prepared as follows.

Liquid silicone (Sylgard 184, Dow Corning, USA) is cast into an appropriately shaped glass mould to produce a layer 1 mm thick and is polymerised at 80°C for 20 minutes.

A sampling mechanism is created by placing a channel-shaped loop object next to two lengths of tubing (normal wall translucent silicone tubing 1 mm x 1 mm, BS2775, Fisherbrand, UK) over the polymerised silicone layer. The two tubing ends extend beyond the silicone backing layer.

Liquid silicone (Sylgard 184, Dow Corning, USA) is further cast into the glass mould to produce a layer 2-3mm thick with a channel shaped void and to encapsulate the inner ends of the two lengths of silicone tubing. The liquid silicone is polymerised at 80°C for 60 minutes.

After silicone polymerisation is complete, the channel-shaped object is removed to reveal a fluid collection channel next to the silicone tubing.

The surface of the silicone backing layer is modified with aminopropyltriethoxysilane (APTES) solution to improve protein immobilisation. The silicone backing layer is immersed in 5% APTES v/v in ethanol overnight at room temperature (5% v/v APTES (3-aminopropyltriethoxysilane, AcroSeal 99%, Acros Organics, Fisher Scientific, UK), 5% H₂O v/v and 90% v/v ethanol (Ethanol, absolute 99%, Fisher Scientific, UK)).

Ports for syringe attachment are glued to the ends of the silicone tubing using cyanoacrylate glue with activator (LOCTITE All Plastics, Henkel, UK. Adhesive base: ethyl cyanoacrylate, activator solvent: heptane).

The channel and tubing are filled with water and the assembly is frozen for 1 hour to prevent cryogel solution entering and polymerising within the channel and the tubing.

Aqueous gelatin solution 6%w/w (Gelatin from cold water fish skin,~45% in H₂O, G7765, Sigma, UK), crosslinked with 0.4%v/v glutaraldehyde (Glutaraldehyde solution, Grade II, 25% in H₂O, G6257, Sigma-Aldrich, UK), is prepared, cast over the silicone backing element and polymerised using a cryopolymerisation process (Julabo ethanol cryobath, -12°C) for 20 hours.

The silicone-cryogel sandwich with incorporated tubing is thawed out at room temperature, removed from the mould and washed with distilled water (x3 times).

To block residual glutaraldehyde within the scaffold, the silicone-cryogel sandwich with incorporated tubing is immersed into 0.5M glycine (glycine, G7126, Sigma, UK) 50mM phosphate-buffered solution (sodium phosphate diabasic, S0876, Sigma, UK) for 20 hours at +4°C.

The scaffold is then washed with distilled water (x3 times) followed by phosphate buffered saline (PBS tablets, BR014G, Oxoid, UK) (x3 times).

The finished scaffold is sealed in polyethylene double sachets with 5ml of PBS to avoid drying out and sterilised with gamma-irradiation (>25kGy, Isotron Ltd., UK).

### EXAMPLE 3

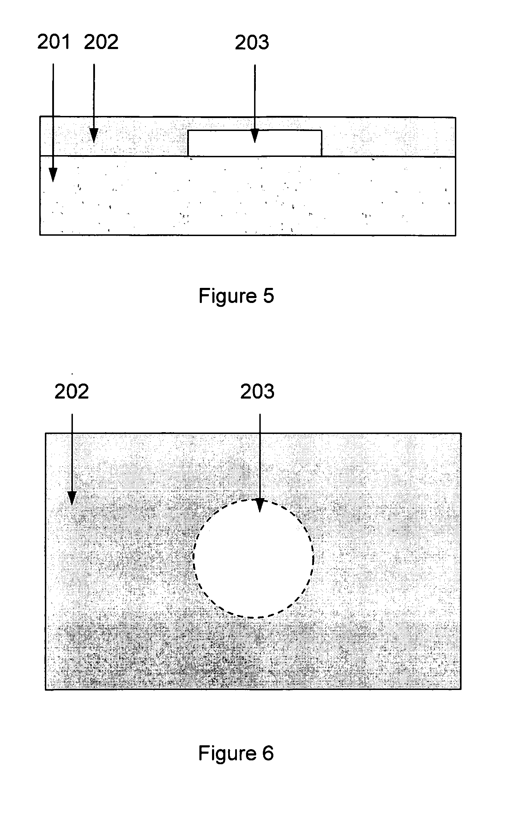

Figures 5 and 6 illustrate a tissue engineering scaffold according to a third embodiment of the invention. In this embodiment, the scaffold comprises a tissue regeneration matrix layer 201 formed from a gelatine cryogel and a protective backing element or layer 202 formed from a silicone material. Formed within the protective backing layer 102 is a sampling bubble 203 which is open on its lower side, i.e. the side facing the gelatine cryogel matrix.

The scaffold of this embodiment is used in a similar manner to the embodiment of Figures 1 to 4 except that the aqueous medium for testing is withdrawn from the sampling bubble by means of a syringe needle. Because the silicone is self sealing, once the needle has been withdrawn, the hole created by the needle seals over to prevent loss of liquid and ingress of contaminants from outside.

The scaffold of Figures 5 and 6 is prepared as follows.

Liquid silicone (Sylgard 184, Dow Corning, USA) is cast into an appropriately shaped glass mould to produce a layer 1 mm thick and is polymerised at 80°C for 20 minutes. A sampling bubble created by placing a bubble-shaped object over the polymerised silicone layer. Liquid silicone (Sylgard 184, Dow Corning, USA) is further cast into the glass mould to produce a layer 2-3mm thick to create a bubble-shaped void. The silicone is polymerised at 80°C for 60 minutes. After silicone polymerisation is complete, the bubble-shaped object is removed to reveal a sampling bubble.

The surface of the silicone is modified with aminopropyltriethoxysilane (APTES) solution to improve protein immobilisation. The assembly is immersed in 5% APTES v/v in ethanol overnight at room temperature (5% v/v APTES (3-Aminopropyltriethoxysilane, AcroSeal 99%, Acros Organics, Fisher Scientific, UK), 5% H₂O v/v and 90% v/v ethanol (Ethanol, absolute 99%, Fisher Scientific, UK)).

The sampling bubble is filled with water and frozen for 1 hour to prevent cryogel solution entering and polymerising within the bubble. Aqueous gelatin solution 6%w/w (Gelatin from cold water fish skin,~45% in H₂O, G7765, Sigma, UK), crosslinked with 0.4%v/v glutaraldehyde (Glutaraldehyde solution, Grade II, 25% in H₂O, G6257, Sigma-Aldrich, UK), is prepared, cast over the silicone backing and polymerised via a cryopolymerisation process (Julabo ethanol cryobath, -12°C) for 20 hours. The silicone-cryogel sandwich with the incorporated sampling bubble is then thawed out at a room temperature, removed from the mould, washed with distilled water x3 times.

To block residual glutaraldehyde within the scaffold, the silicone-cryogel sandwich with incorporated sampling bubble is immersed into 0.5M glycine (Glycine, G7126, Sigma, UK) 50mM phosphate-buffered solution (Sodium phosphate diabasic, S0876, Sigma, UK) for 20hrs at +4°C.

The tissue engineering scaffold is then washed with distilled water (x3 times) followed by phosphate buffered saline (PBS tablets, BR014G, Oxoid, UK) (x3) times.

T he completed tissue engineering scaffold is then sealed in polyethylene double sachets with 5ml of PBS to avoid drying out and sterilised with gamma-irradiation (>25kGy, Isotron Ltd., UK).

### EXAMPLE 4

For the scaffolds of the invention to work effectively, biomarker molecules within the tissue regeneration matrix must be capable of migrating onto the sampling cavities so that they can be detected and, where necessary, quantified. If there is no diffusion of biomarkers into the cavities, then the diagnostic usefulness of the scaffolds is restricted.

Therefore, scaffolds corresponding to those described in Examples 1 and 2, but of circular rather than rectangular shape, were tested to assess whether molecules of different sizes would pass through the scaffolds and into the collection channels and to assess the extent to which molecules of interest were retained in the channels. Tests were carried out by immersing the scaffolds in solutions of various dyestuffs as well as solutions of proteins. In some cases, where stated, human cells were grown into the scaffolds before carrying out the tests.

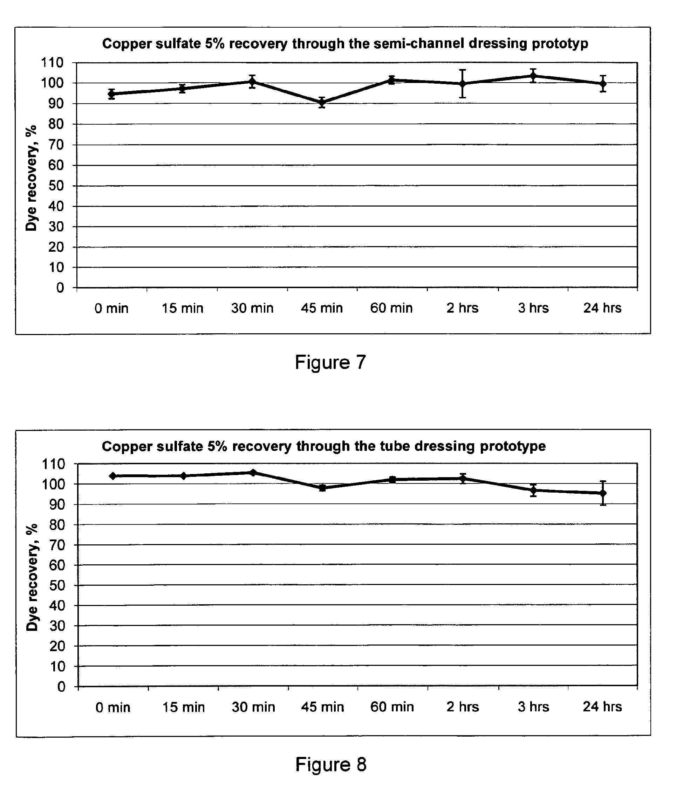

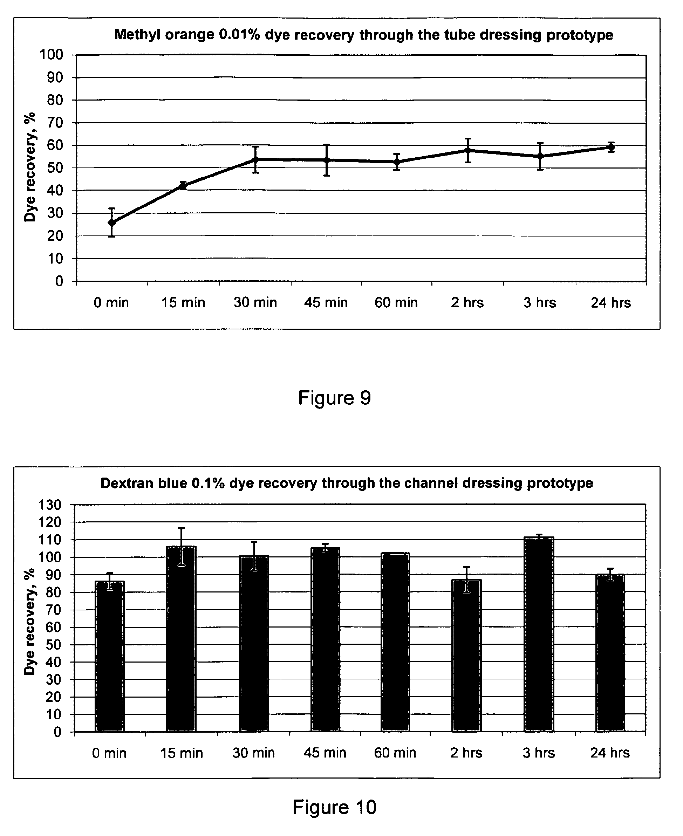

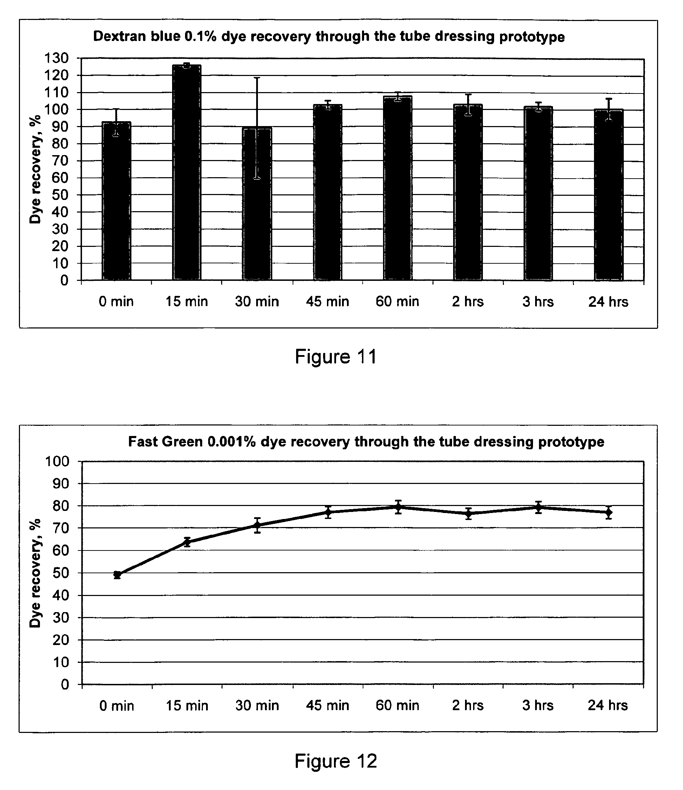

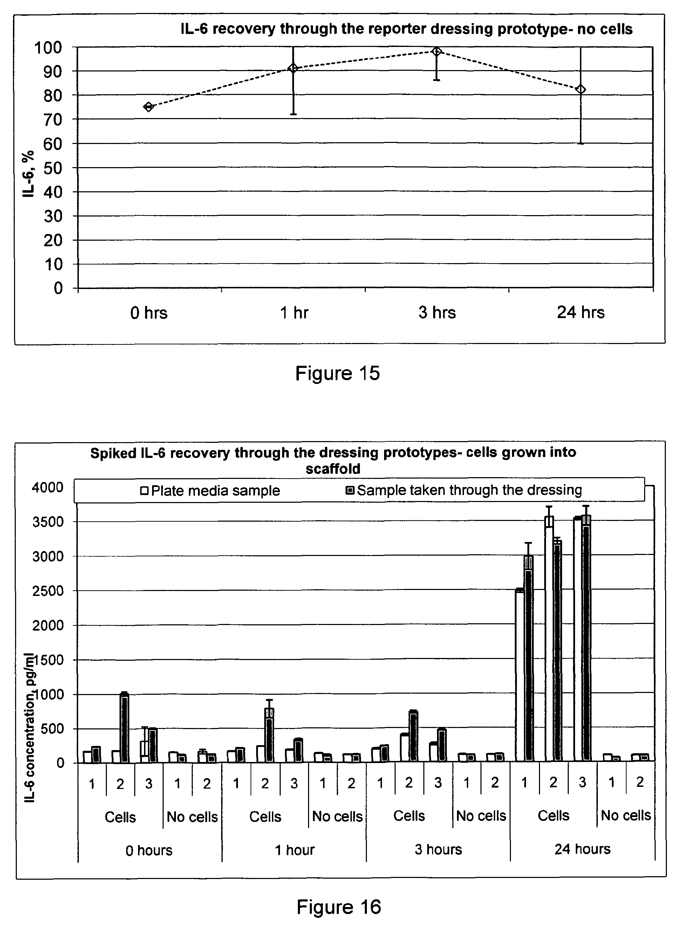

The protocols for the tests are described below and the results are shown in Figures 7 to 16.

In the paragraphs below, and in Figures 7 to 16, the scaffold based on Example 1 is referred to as the "tube dressing" and the scaffold based on Example 2 is referred to as the "channel dressing".

### Materials:

The following test substances were used:
   Dyes or coloured substances:
Copper (II) sulphate - molecular weight 159.61 (Sigma-Aldrich catalogue number 209201)
Methy orange dye - molecular weight 327.33 (BDH catalogue number 20065) Bromophenol blue dye - molecular weight 669.96 (Surechem Prodcuts Ltd, catalogue number B7722)
Fastgreen FCF dye - molecular weight 808.85 (Sigma-Aldrich catalogue number 861154)
Blue dextan dye - molecular weight 2,000,000 (Sigma-Aldrich catalogue number D5751)
Proteins:
   Bovine serum albumin (Albumin standard 2mg/mL, Thermo Scientific Pierce, Fisher Scientific catalogue number PN23209) assessed using Coomassie Plus protein assay kit (Thermo Scientific Pierce, Fisher Scientific catalogue number PN23236)
   MMP-9 Recombinant human protein (Invitrogen, Fisher Scientific catalogue number AFRP75653 assessed with MMP-9 ELISA kit, human (Invitrogen, Fisher Scientific catalogue number VXKHC3061)
   IL-6 Recombinant human protein (Invitrogen, Fisher Scientific catalogue number VXPHC0065) assessed with IL-6 ELISA kit, human (Invitrogen, Fisher Scientific catalogue number VXKAC1261)

### Human cells

Normal human skin fibroblasts SKF276 at early passages are seeded into the scaffolds.

### Flushing solutions:

For the *in vitro* studies with dye molecules, distilled water or phosphate buffered saline was used as the flushing solvent.

For the *in vitro* studies with protein molecules (cytokines), phosphate buffered saline containing 10% bovine serum albumin was used so as to prevent protein deactivation.

For the *in vitro* studies with primary human skin cells, the flushing solvent contained DMEM (Dulbecco's Modified Eagle Medium) with 10% bovine serum albumin.

### Methods:

### Dye solutions

The channel or tube dressings were immersed in dye solution (20 ml). At time intervals of 0, 15, 30, 45 and 60 minutes and 2, 3 and 24 hours, samples were withdrawn through the ports using a syringe and were dispensed into 96-well plates where they were analysed using a spectrophotometer plate reader set to the relevant wavelength for each dye.

The results, defined in terms of the percentage recovery of dye from the scaffold compared to a control plate, are shown in Figures 7 to 12.

### Proteins:

The channel or tube dressings were treated with solutions of the proteins in analogous manner to the treatment with dye solutions are described above except that the concentrations of proteins in the sampled solutions was tested by the trest methods described in the Materials section above.

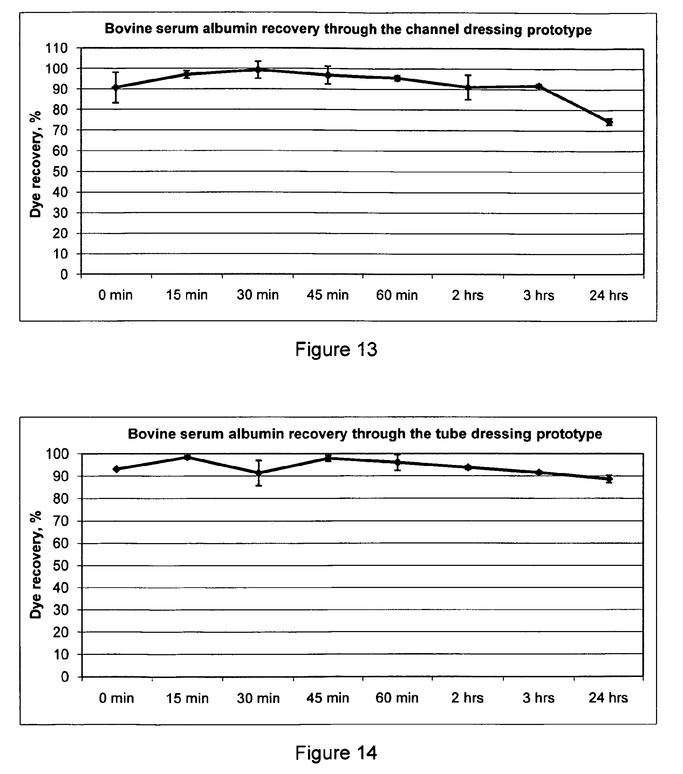

The results are shown in Figures 13 to 16.

### Human fibroblasts:

Normal human skin primary fibroblasts (SKF276) were thawed out at early passage (University of Brighton cell cryobank) and subcultured on plastic to recover and to be screened for microbial contamination.

The scaffolds were sterilised with γ-irradiation at 25kGy (Isotron Ltd., UK).

The recovered cells were harvested, concentrated and aseptically seeded into the test scaffolds in a dropwise manner, allowed to anchor for two hours. Fibroblast growth medium (DMEM + 10% FCS), Gibco, Invitrogen) was then added and the cell-seeded scaffolds were incubated at 37 °C under an atmosphere containing 5% CO₂ in a humidified incubator.

### Results and conclusions:

The results demonstrate that the scaffolds of the invention allow the passage of molecules of different sizes through the scaffolds although some retention of the dye or protein was observed depending on the nature of the molecule. A non-dye protein molecule (bovine serum albumin) and blue dextran as a large molecule dye showed 90-100% recovery after passage through the scaffolds after a very short saturation time. This suggests that large molecules can pass freely through the scaffold and are therefore available for analysis and quantification.

### Equivalents

It will readily be apparent that numerous modifications and alterations may be made to the specific embodiments of the invention described above without departing from the principles underlying the invention. All such modifications and alterations are intended to be embraced by this application.

## Claims

1. A tissue engineering scaffold for application to a wound, the tissue engineering scaffold comprising a tissue regeneration matrix layer for contacting the wound and a protective backing element bonded to the tissue regeneration matrix layer, the tissue regeneration matrix layer being formed from a porous biodegradable material capable of taking up cellular material from the wound and supporting tissue generation from the cellular material, and the protective backing element being formed from a water-impermeable material;
wherein the protective backing element is provided with at least one cavity therein containing an aqueous medium, the channel or cavity being in fluid communication with the tissue regeneration matrix layer so that one or more biomarker substances from the tissue regeneration matrix layer can diffuse into the aqueous medium the cavity;
and wherein the protective backing element is configured to allow aqueous medium to be withdrawn from the cavity for analysis.

2. A tissue engineering scaffold according to claim 1 wherein the cavity is a channel which is connected to a port.

3. A tissue engineering scaffold for application to a wound according to claim 1, the tissue engineering scaffold comprising a tissue regeneration matrix layer for contacting the wound and a protective backing element bonded to the tissue regeneration matrix layer, the tissue regeneration matrix layer being formed from a porous biodegradable material capable of taking up cellular material from the wound and supporting tissue generation from the cellular material, and the protective backing element being formed from a water-impermeable material;
wherein the protective backing element is provided with at least one channel therein, the channel being connected to a port through which an aqueous medium can be introduced into the channel or withdrawn therefrom, and wherein the channel is in fluid communication with the tissue regeneration matrix layer so that one or more biomarker substances from the tissue regeneration matrix layer can diffuse into aqueous medium present in the channel and subsequently be withdrawn though the port for analysis.

4. A tissue engineering scaffold according to claim 1 wherein the takes the form of a bubble which can be accessed by syringe needle to extract the aqueous medium.

5. A tissue engineering scaffold for application to a wound according to claim 4, the tissue engineering scaffold comprising a tissue regeneration matrix layer for contacting the wound and a protective backing element bonded to the tissue regeneration matrix layer, the tissue regeneration matrix layer being formed from a porous biodegradable material capable of taking up cellular material from the wound and supporting tissue generation from the cellular material, and the protective backing element being formed from a water-impermeable material;
wherein the protective backing element is provided with at least one cavity therein containing an aqueous medium, the channel or cavity being in fluid communication with the tissue regeneration matrix layer so that one or more biomarker substances from the tissue regeneration matrix layer can diffuse into the aqueous medium the cavity;
and wherein the protective backing element is formed from a self sealing material through which a syringe needle can be pushed so that aqueous medium can be withdrawn from the cavity for analysis.

6. A tissue engineering scaffold according to any one of claims 1 to 5 wherein the backing element is formed from a silicone polymer.

7. A tissue engineering scaffold according to any one of claims 1 to 6 wherein the tissue regeneration scaffold is formed from a biodegradable biocompatible gel material.

8. A tissue engineering scaffold according to claim 7 wherein the biodegradable biocompatible gel material comprises collagen or gelatine.

9. A tissue engineering scaffold according to claim 8 wherein the biodegradable biocompatible gel material comprises gelatine.

10. A tissue engineering scaffold according to any one of claims 7 to 9 wherein the gel material comprises a cryogel.

11. A method of monitoring the progress of healing of a wound, which method comprises one or more cycles, each of which cycles comprises the steps applying to the wound a tissue engineering scaffold as defined in any one of the preceding claims, filling the cavity with an aqueous medium (preferably a physiologically acceptable aqueous medium such as physiological saline), and where necessary closing any ports to prevent loss of the aqueous medium, leaving the aqueous medium in the channel for a defined period, withdrawing the aqueous medium from the cavity, and testing the aqueous medium to identify and optionally quantify one or more selected biomarkers in the aqueous medium.
